# EUROPÄISCHE PATENTSCHRIFT

(11) **EP 1 923 008 B1**
(45) Veröffentlichungstag und Bekanntmachung des Hinweises auf die Patenterteilung: **24.06.2009**
(21) Anmeldenummer: 06023960.5
(22) Anmeldetag: 17.11.2006
(51) Int. Cl.: A61B 17/22, A61B 17/00, A61B 17/32

(54) **Gefäßreiniger zur Reinigung von Blutgefäßen**
Vessel cleaner for cleaning blood vessels
Purificateur de vaisseaux pour la purification de vaisseaux sanguins

(43) Veröffentlichungstag der Anmeldung: 21.05.2008
(73) Patentinhaber: Hartmann, David, 97980 Bad Mergentheim (DE)
(72) Erfinder: Hartmann, David, 97980 Bad Mergentheim (DE)
(74) Vertreter: Riedel, Peter

(56) Entgegenhaltungen:
- WO-A-96/34573
- DE-A1- 10 161 958
- FR-A- 1 604 860
- US-A- 5 376 100
- US-A1- 2001 051 766
- US-A1- 2003 214 579
- NAKAJIMA N: "SMALL WONDER" LOOK JAPAN, SINGAPORE, SG, August 1993 (1993-08), Seiten 22-23, XP000749842

## Beschreibung

Die Erfindung betrifft einen Gefäßreiniger zur Reinigung von Blutgefäßen.

Gefäßerkrankungen spielen in der Volksgesundheit eine bedeutende Rolle. Ernährungsmäßige und medizinische Präventivmaßnahmen können nur eingeschränkt verhindern, daß sich in Blutgefäßen Kalk (Arteriosklerose), Streßhormone (Adrenalin) und Blutfette (Cholesterin) ablagern, was die Blutversorgung des Organismus beeinträchtigt.

Eine mechanische Reinigung der Gefäßwände von den vorgenannten Ablagerungen ist nur eingeschränkt möglich. Mit eingeführten Sonden lassen sich nur begrenzte Gefäßbereiche beeinflussen Dokument US-A-5376100 offenbart eine Sonde mit expandierbaren Segmenten. Es wurde bereits vorgeschlagen, frei in der Blutbahn bewegliche Gefäßreiniger einzusetzen, die als Pfropfen ausgebildet sind Siehe z.B. DE-A-10161958 und WO-A-9634573. Am Pfropfen bildet sich ein Staudruck aus, über den der Pfropfen durch das Blutgefäß gedrückt wird. Außenseitige mechanische Reinigungsmittel sollen die Ablagerungen von den Gefäßwänden entfernen. Die Stauwirkung des Pfropfens behindert jedoch die Blutzirkulation, was insbesondere beim Hängenbleiben des Pfropfens nachteilig ist. Problematisch ist auch, daß die mechanische Reinigung Spuren beispielsweise in Form von Fissuren oder dergleichen an den Gefäßwänden hinterläßt, die eine erneute Bildung von Ablagerungen begünstigen.

Eine weitere Schwierigkeit besteht in den deutlich variierenden Innendurchmessern der Blutgefäße. Diese reichen von etwa 1 µm bei Kapillargefäßen bis hin zu etwa 2 cm in der Aorta. Mit einem der vorgenannten Pfropfen ist nur ein eingeschränkter, ausgewählter Durchmesserbereich der Blutgefäße behandelbar.

Der Erfindung liegt die Aufgabe zugrunde, einen Gefäßreiniger anzugeben, mit dem eine wirkungsvolle, zuverlässige und dauerhafte Beseitigung von Gefäßablagerungen möglich ist.

Diese Aufgabe wird durch einen Gefäßreiniger mit den Merkmalen des Anspruchs 1 gelöst.

Es wird ein Gefäßreiniger zur Reinigung von Blutgefäßen vorgeschlagen, der eine rohrförmige Reinigungshülse umfaßt. Die Reinigungshülse ist außenseitig mit mechanischen Reinigungsmitteln versehen, die insbesondere als Borsten ausgebildet sind. Des weiteren ist die Reinigungshülse bezogen auf eine Umfangsrichtung des Gefäßreinigers in Segmente aufgeteilt, wobei die Segmente in der Umfangsrichtung im Abstand zueinander einstellbar sind. Der Gefäßreiniger weist einen in seiner Axialrichtung verlaufenden Strömungskanal für Blut auf. Der vorgenannte Gefäßreiniger wird betrieben, indem eine Durchströmung des Strömungskanals mit Blut zugelassen wird, wobei der Gefäßreiniger infolge seines beim Durchtritt des Blutstromes entstehenden Durchströmungswiderstandes durch das Blutgefäß hindurch bewegt wird. Unabhängig von seiner Bewegungsgeschwindigkeit und selbst im blockierten Zustand läßt der Strömungskanal den Durchtritt eines Blutstromes zu. Die Blutzirkulation ist durch die Anwendung des erfindungsgemäßen Gefäßreinigers nicht oder nicht wesentlich beeinträchtigt. Dennoch erfolgt eine selbsttätige Bewegung des Gefäßreinigers entlang der Blutbahn zusammen mit dem Blutstrom. Die außenliegenden mechanischen Reinigungsmittel führen infolge dieser selbsttätigen Bewegung eine ebenso selbsttätige Reinigung der Gefäßwände durch. Eine Einflußnahme von außen ist nicht erforderlich.

Die in der Umfangsrichtung im Abstand zueinander einstellbaren Segmente erlauben eine Anpassung an verschiedene Gefäßdurchmesser, so daß ein großer Anteil der Blutgefäße wirkungsvoll gereinigt werden kann. Insbesondere mit den Mitteln der Nanotechnologie kann der erfindungsgemäße Gefäßreiniger hinreichend klein ausgeführt sein, so daß er beispielsweise mit einem Außendurchmesser von 1 µm selbsttätig durch Kapillargefäße geführt wird. Mit geeigneten, weiter unten näher beschriebenen Mitteln kann durch Vergrößerung des Abstandes der Segmente in Umfangsrichtung ein vergrößerter Außendurchmesser hergestellt werden, der auch zur Reinigung von größeren Blutgefäßen geeignet ist. Es ist denkbar, daß damit sogar ein Durchmesser von mehr als 2 cm erreichbar ist, so daß mit demselben Gefäßreiniger sämtliche Gefäße an den feinsten Kapillaren bis hin zur Aorta behandelt werden können. Insbesondere ist die Möglichkeit geschaffen, den Gefäßreiniger dauerhaft im Blutkreislauf zu belassen. Infolge seiner selbsttätigen Durchmesseranpassung zirkuliert er ständig im Blutkreislauf. Neben der Abtragung von bereits gebildeten Ablagerungen an den Innenwänden der Blutgefäße wird die Bildung von erneuten Ablagerungen gleich zu Beginn verhindert oder zumindest eingeschränkt.

In vorteilhafter Weiterbildung sind Spalte zwischen den Segmenten der Reinigungshülse keilförmig in einer Radialrichtung nach innen geöffnet ausgebildet. Insbesondere prismatische Füllstäbe sind zur Auffüllung der Spalte innenseitig der Reinigungshülse bevorratet. Sofern sich der Gefäßreiniger in einem Gefäßabschnitt mit sich erweiterndem Radius bewegt, vergrößern die Segmente selbsttätig ihren Abstand zueinander, wobei die Spalte zwischen den Segmenten breiter werden. Diese Spalte werden in weiter unten näher beschriebener Weise durch die Füllstäbe aufgefüllt, so daß die Reinigungshülse ihre im wesentlichen zylindrische Grundform beibehält. Von der Kreisform abweichende Querschnittsformen des Gefäßes können durch die Füllstäbe ausgeglichen werden, so daß die mit den Reinigungsmitteln versehene Umfangskontur des Gefäßreinigers zuverlässig an den Gefäßwänden anliegt. Umgekehrt drücken die Gefäßwände bei entlang dem Strömungsweg sich verengendem Querschnitt die Segmente gegeneinander. Die keilförmige Öffnung der Spalte führt dazu, daß die Füllstäbe selbsttätig in den Innenraum der Reinigungshülse hineingedrückt werden, ohne die verengende Bewegung im Spalt zu behindern. Insgesamt ist erreicht, daß sowohl eine Querschnittsvergrößerung als auch eine Querschnittsverkleinerung selbsttätig erfolgt. Eine flächige Anlage der Umfangkontur der Reinigungshülse an der Gefäßinnenwand ist unabhängig von deren Durchmesserverlauf dauerhaft sichergestellt. In Verbindung mit den daran angebrachten mechanischen Reinigungsmitteln ist eine zuverlässige Reinigungswirkung selbst bei großen Durchmesserschwankungen möglich.

In bevorzugter Weiterbildung sind die Segmente aus einem permanentmagnetischen Material gebildet, wobei die Segmente eine untereinander gleiche, in der Radialrichtung des Gefäßreinigers verlaufende magnetische Polarisierungsrichtung aufweisen. An jedem Spalt zwischen zwei angrenzenden Segmenten liegen sich in der Umfangsrichtung gleiche magnetische Pole gegenüber, die eine Abstoßungskraft in der Umfangsrichtung zwischen zwei benachbarten Segmenten bewirkt. Dies ermöglicht ein entsprechendes Betriebsverfahren, bei dem eine selbsttätige Aufweitung des Abstandes zwischen den magnetischen Segmenten durch die Magnetische Abstoßungskräfte vorgenommen wird. Beim Einsatz körperverträglicher, dauermagnetischer Materialien ist mit einfachen Mitteln eine selbsttätige und im wesentlichen verschleißfreie Durchmesseranpassung ermöglicht, die einen dauerhaften, gegebenenfalls lebenslangen Verbleib des Gefäßreinigers im Blutkreislauf zuläßt. Die magnetische Aufweitung erfordert nur geringe Materialquerschnitte. Die Segmente können sehr dünnwandig ausgeführt sein und sind deshalb insbesondere zur Ausführung in der Nanotechnologie geeignet. Eine zuverlässige Funktion kann selbst bei solchen Wandstärken sichergestellt werden, die für eine Ausführung des Gefäßreinigers mit einem kleinsten Durchmesser von etwa 1 µm erforderlich sind.

Die Füllstäbe sind zweckmäßig aus einem weichmagnetischen Material gefertigt. Im Zustand ihrer Bevorratung haften sie infolge der magnetischen Anziehungskräfte der Hülsensegmente an deren Innenseite. Ausgehend hiervon können sie in weiter unten näher beschriebener Weise in die sich öffnenden Spalte und auch von dort wieder zurück bewegt werden. Infolge ihres Kontaktes mit den permanentmagnetischen Segmenten nehmen sie selbst einen magnetisierten Zustand an, so daß sie beim Auffüllen des Spaltes zu einem Teil der einzelnen Segmente werden. Der Mechanismus des zuvor beschriebenen selbsttätigen Aufweitens infolge der magnetischen Abstoßungskräfte sowie die selbsttätige Verringerung des Spaltmaßes bleibt auch bei sehr großen Spaltmaßen erhalten. Der maximale Durchmesser des Gefäßreinigers kann infolge der sukzessiven Spaltauffüllung durch die Füllstäbe mehrere Größenordnungen über dem minimalen Durchmesser des Gefäßreinigers liegen.

Die Füllstäbe sind dabei zweckmäßig mit elastisch verformbaren mechanischen Reinigungsmitteln versehen. Hierdurch ergänzen sie die bereits an der Umfangsseite der Hülsensegmente angebrachten mechanischen Reinigungsmittel unabhängig vom jeweils eingestellten Spaltmaß. Dort wo die Füllstäbe aneinander bzw. an den Segmenten anliegen, werden die elastisch verformbaren Reinigungsmittel elastisch eingedrückt. Je nach Position und Lage, die die Füllstäbe in den Spalten einnehmen, richten sich die mechanischen Reinigungsmittel beispielsweise in Form von Borsten aufgrund ihrer elastischen Eigenschaft selbsttätig auf und beteiligen sich am Reinigungsprozeß.

In vorteilhafter Weiterbildung ist im Strömungskanal ein Rotor angeordnet, zu dessen Antrieb der durchtretende Blutstrom vorgesehen ist. Die Drehung des Rotors erzeugt im durchtretenden Blutstrom einen Drall, der seinerseits auf die innenseitig der Hülsensegmente bevorrateten Füllstäbe einwirkt. Im entsprechenden erfindungsgemäßen Verfahren wird eine Bewegung der Füllstäbe in der Umfangsrichtung durch Drehung des Rotors herbeigeführt, wobei sich die Füllstäbe unter Einfluß der wirkenden Magnetkräfte und der auf ihrer gekrümmten Bahn wirkenden Fliehkräfte in die Spalte zwischen den Segmenten setzen. Wie zuvor beschrieben, wird der erfindungsgemäße Gefäßreiniger zwar mit dem Blutstrom fortgetragen. Da jedoch ein Blutstrom durch den Strömungskanal hindurchtritt, ist die Bewegungsgeschwindigkeit des Gefäßreinigers langsamer als die Strömungsgeschwindigkeit des Blutes. Die Differenzgeschwindigkeit wird als Antriebsenergie für die Drehbewegung des Rotors genutzt. Der im Blutstrom erzeugte Drall schwemmt die Füllstäbe in die sich aufweitenden Spalte. Bei geeignetem Durchmesser des Rotors kann auch ein mechanischer Kontakt zwischen dem Rotor und den Füllstäben herbeigeführt werden, der eine entsprechende Bewegung der Füllstäbe herbeiführt.

In zweckmäßiger Weiterbildung ist der Rotor zumindest teilweise aus einem permanentmagnetischen Material gebildet, wobei zumindest Spitzen von Rotorschaufeln des Rotors, bevorzugt auch eine Nabe des Rotors eine in der Radialrichtung verlaufende magnetische Polarisierungsrichtung aufweisen, die der magnetischen Polarisierung der Segmente entgegengesetzt ist. Hierdurch lassen sich mehrere Funktionen gleichzeitig erfüllen: Die im Vergleich zur Polarisierungsrichtung der Hülsensegmente entgegengesetzte Polarisierung der Rotorspitzen, gegebenenfalls auch der Nabe führt zu radial wirkenden Abstoßungskräften. Diese Abstoßungskräfte können für eine kontaktlose Zentrierung und Drehlagerung des Rotors genutzt werden, so daß auf eine eigenständige Lagerung des Rotors gegenüber der Reinigungshülse verzichtet werden kann. Insbesondere bei einer Ausführung mit im Durchmesser variablen Rotorblättern wird eine radiale Begrenzung einer selbsttätigen Größenanpassung der Rotorblätter erzeugt. Diese können sich infolge ihrer Rotationsbewegung und der dabei auftretenden Fliehkräfte nur so weit in der radialen Richtung ausdehnen, wie es die Abstoßungskräfte an den Blattspitzen zulassen. Ein blockierender Kontakt mit der nicht mitdrehenden Reinigungshülse ist zuverlässig vermieden. Eine freie Drehbarkeit des Rotors ist bei variablem Durchmesser sichergestellt.

Der Rotor weist zweckmäßig Rotorschaufeln mit selbsttätig einstellbarem Radius auf, die bevorzugt aus in der Radialrichtung angeordneten Teleskopelementen gebildet sind, wobei zumindest ein Teil der Teleskopelemente quer zur Radialrichtung permanent magnetisch polarisiert ist. Die quer verlaufende Polarisierung führt dazu, daß benachbarte Teleskopsegmente aneinander haften. Durch eine geeignete Anzahl von Teleskopstäben, die nebeneinander liegen und dabei aneinander haften, lassen sich geeignete Querschnitte und damit Strömungsprofile der Rotorschaufeln realisieren. Die quer verlaufende Polarisierung führt darüber hinaus dazu, daß sich die einzelnen Teleskopelemente in der Radialrichtung gegeneinander abstoßen. Zusätzlich zur wirkenden Fliehkraft soll diese Abstoßung eine selbsttätige Vergrößerung des Rotorschaufel-Radius mit der zuvor beschriebenen Begrenzung bewirken. Die Begrenzung infolge der magnetischen Abstoßungskräfte wird auch genutzt, um bedarfsweise eine selbsttätige Verringerung des Schaufeldurchmessers herbeizuführen. Wenn die Segmente der Reinigungshülse selbsttätig durch die Innenwände des Blutgefäßes zusammengedrückt werden, drücken sie über die Abstoßungkräfte auch die Schaufelspitzen nach innen. Der wirksame Durchmesser des Rotors wird also selbsttätig an den jeweils wirksamen Durchmesser der Reinigungshülse angepaßt, so daß seine angestrebte Wirkung auf die Verteilung der Füllstäbe im gesamten vorgesehenen Durchmesserbereich sichergestellt ist.

In einer bevorzugten Weiterbildung ist stirnseitig der Reinigungshülse eine radial aufweitbare, insbesondere permanentmagnetisch polarisierte Begrenzungseinrichtung zur Begrenzung einer radialen Aufweitung der Segmente vorgesehen. Dies trägt dem Umstand Rechnung, daß die konstruktive Ausgestaltung des Gefäßreinigers nur einen bestimmten maximalen Durchmesser zuläßt. Sofern der Gefäßreiniger in einen Gefäßbereich gelangt, dessen Durchmesser größer als der maximal erzielbare Durchmesser des Gefäßreinigers ist, verhindert die Begrenzungseinrichtung durch magnetische Anziehungskräfte, daß sich die einzelnen Segmente und die zwischenliegenden Füllstäbe voneinander lösen. Insbesondere beim Eintritt des Gefäßreinigers in eine Herzkammer fällt die äußere Stützwirkung der Gefäßinnenwand fort. Die Begrenzungseinrichtung stellt sicher, daß sich der Gefäßreiniger auch beim Fehlen dieser Stützwirkung nicht in seine Einzelteile zerlegt. Vorteilhaft ist die Begrenzungseinrichtung durch die weitere unten näher beschriebene Versiegelungseinrichtung bzw. deren Turbinenschaufeln gebildet. Hierbei kann auf eine separate Begrenzungseinrichtung verzichtet werden, wodurch der Aufbau des erfindungsgemäßen Gefäßreinigers einfach bleibt.

In einer vorteilhaften Ausführungsform ist auf einer bezogen auf die Bewegungsrichtung rückwärtigen Stirnseite der Reinigungshülse eine Versiegelungseinrichtung für eine Innenwand des Blutgefäßes angeordnet, wobei die Versiegelungseinrichtung ein radial inneres Depot für ein Versiegelungsmittel sowie eine zum Antrieb vom durchtretenden Blutstrom vorgesehene Turbine umfaßt, und wobei die Turbine in der Radialrichtung verlaufende Förderkanäle für das Versiegelungsmittel aufweist. Der durch die Turbine hindurchtretende Blutstrom versetzt die Versiegelungseinrichtung in Drehung. Im zugehörigen erfindungsgemäßen Betriebsverfahren wird eine Förderung des Versiegelungsmittels vom Depot durch die Förderkanäle hindurch radial nach außen infolge der dabei wirkenden Fliehkräfte vorgenommen. Auf diese Weise gelangt das Versiegelungsmittel unter Ausnutzung der Bewegungsenergie des Blutstromes selbsttätig und ohne äußere Beeinflussung vom Depot zur Innenwand des Blutgefäßes. Beschädigungen in Form von Fissuren oder dergleichen, die gegebenenfalls von der in Bewegungsrichtung vorauseilenden Reinigungshülse hervorgerufen wurden, können ausgeglichen bzw. geheilt werden. Die Tendenz zur erneuten Bildung von Ablagerungen an der Gefäßwand ist verringert.

Die Turbine und ihre Turbinenschaufeln sind bevorzugt vergleichbar zum vorstehend beschriebenen Rotor und dessen Schaufeln insbesondere im Hinblick auf die teleskopartige Ausgestaltung und die Magnetisierung aufgebaut. Es erfolgt eine selbsttätige Durchmesseranpassung. Ein beabsichtigter funktionaler Unterschied liegt jedoch darin begründet, daß die Turbinenschaufeln nicht radial innenseitig, sondern stirnseitig der Reinigungshülse angeordnet sind. Die in umgekehrter radialer Richtung magnetisierten Schaufelspitzen der Turbine erfahren von den magnetisierten Hülsensegmenten eine axiale Anziehungskraft. Insbesondere bei großen Arbeitsdurchmessern ist diese axiale Anziehungskraft geeignet, die Blattspitzen der Turbinenschaufeln stirnseitig an die Reinigungshülse anzupressen. Die mit entsprechenden Anschlägen versehenen Teleskopelemente der Turbinenschaufeln können nur einen vorgegebenen maximalen Durchmesser erreichen. Infolge ihrer stirnseitigen Anlage an den Hülsensegmenten werden die Hülsensegmente in radialer Richtung gehalten, wodurch die vorstehend beschriebene Begrenzungseinrichtung für die Segmente gebildet ist.

In diesem Zustand wird der Gefäßreiniger vom Blutstrom fortgetragen, ohne daß ein Kontakt zur Gefäßwand besteht. Hierbei fehlt eine axiale Durchströmung des Gefäßreinigers, in dessen Folge der innere Rotor und/oder die Turbine der Versiegelungseinrichtung ohne Antriebsenergie sind. Die Freigabe des Versiegelungsmittels ist unterbrochen. Beim erneuten Eintritt des Gefäßreinigers in einen Gefäßbereich mit hinreichend kleinem Durchmesser wird der Gefäßreiniger durch seinen Kontakt mit den Gefäßwänden abgebremst. Es baut sich erneut eine axiale Durchströmung mit dem Blutstrom auf, die geeignet ist, die bremsenden Anlagekräfte der Turbinenschaufelspitzen zu überwinden und damit die Turbine in Drehung zu versetzen. Die vorstehend beschriebene Funktion stellt sich wieder ein. Gleichzeitig ist die radiale Größenveränderung der Reinigungshülse wieder freigegeben.

Die Turbinenschaufeln sind in bevorzugter Weiterbildung radial außenseitig gerundet ausgeführt und weisen dort Hohlborsten zur Anbringung des Versiegelungsmittels an die Innenwand des Blutgefäßes auf. Die Rundung und eine hinreichend weiche Ausbildung der Hohlborsten vermeiden eine zusätzliche mechanische Belastung der Gefäßinnenwand. Gleichzeitig stellen die Hohlborsten eine gleichmäßige Verteilung des Versiegelungsmittels an der Gefäßinnenwand sicher.

In zweckmäßiger Weiterbildung sind zwei im wesentlichen identisch ausgebildete Versiegelungseinrichtung an je einer Stirnseite der Reinigungshülse spiegelsymmetrisch zueinander angeordnet. Hierdurch kann der Gefäßreiniger seine Reinigungs- und Versiegelungsfunktion unabhängig von seiner Bewegungsrichtung ausführen. Beispielsweise nach einem frei schwimmenden Zustand in der Herzkammer kommt es nicht darauf an, mit welcher seiner Stirnseiten der Gefäßreiniger zuerst wieder in das nächste Gefäß eintritt. Auf entsprechende Steuer- und Leitmittel kann verzichtet werden. Durch geeignete, weiter unten näher beschriebene Maßnahmen kann sichergestellt werden, daß von den beiden Versiegelungseinrichtungen nur jeweils die in Bewegungsrichtung hintere Versiegelungseinrichtung aktiv ist, während die gegenüberliegende, vordere Versiegelungseinrichtung abgebremst, blockiert oder in anderer Weise außer Funktion gesetzt ist. Dadurch ist sichergestellt, daß eine Versiegelung erst im direkten Anschluß an den Reinigungsvorgang, nicht jedoch schon vorab erfolgt.

Ein Ausführungsbeispiel der Erfindung ist im Folgenden anhand der Zeichnung näher beschrieben. Es zeigen:
- Fig. 1: eine schematische Perspektivdarstellung eines erfindungsgemäßen Gefäßreinigers mit einer mittigen Reinigungshülse und mit zwei stirnseitig angeordneten Versiegelungseinrichtungen;
- Fig. 2: eine perspektivische Detaildarstellung der aus Segmenten gebildeten Reinigungshülse nach Fig. 1 mit einem innenliegenden Rotor;
- Fig.3: eine vergrößerte Detaildarstellung der Einzelheit III nach Fig. 2 im Bereich eines Spaltes mit einem zwischenliegenden Füllstab;
- Fig. 4: eine schematische Ausschnittsdarstellung des Rotors nach Fig. 2 mit Einzelheiten seiner magnetischen Polarisierung;
- Fig. 5: eine schematische Ausschnittsdarstellung der Versiegelungseinrichtung nach Fig. 1 mit Einzelheiten zur Förderung des Versiegelungsmittels und der magnetischen Polarisierung;
- Fig. 6: eine schematische Längsschnittdarstellung des Gefäßreinigers nach Fig. 1 zur Darstellung der Wechselwirkung einzelner Komponenten untereinander.

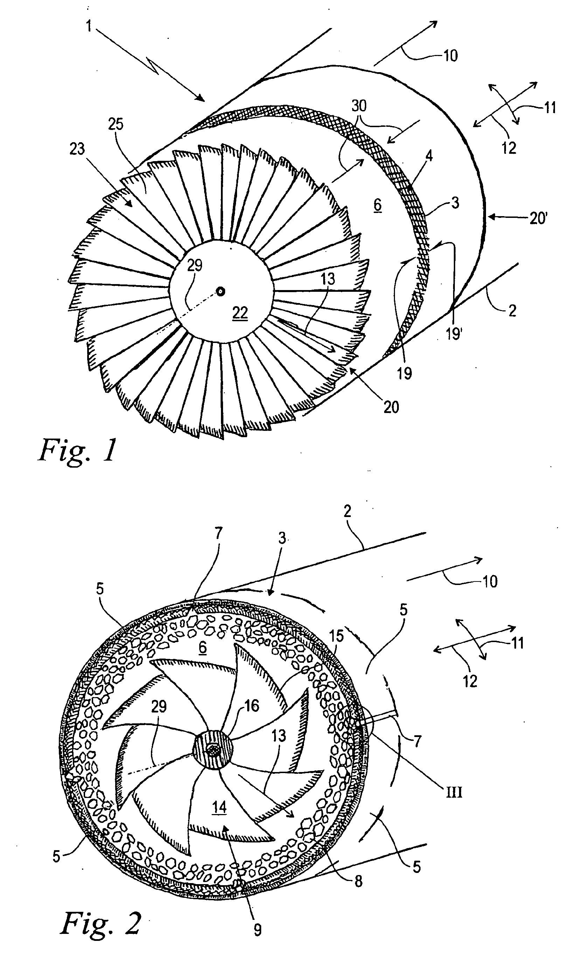

Fig. 1 zeigt in schematischer Perspektivdarstellung ein Ausführungsbeispiel eines erfindungsgemäßen Gefäßreinigers 1 in einem angedeuteten Blutgefäß 2. Der Gefäßreiniger 1 umfaßt eine weiter unten näher beschriebene Reinigungshülse 3, an dessen beiden Stirnseiten 19, 19' je eine Versiegelungseinrichtung 20, 20' angeordnet ist. Die beiden Versiegelungseinrichtungen 20, 20' sind der besseren Übersichtlichkeit halber mit axialem Abstand zur Reinigungshülse 3 gezeigt, grenzen jedoch im betriebsfertigen Zustand entsprechend Pfeilen 30 direkt an die Stirnseite 19, 19' an.

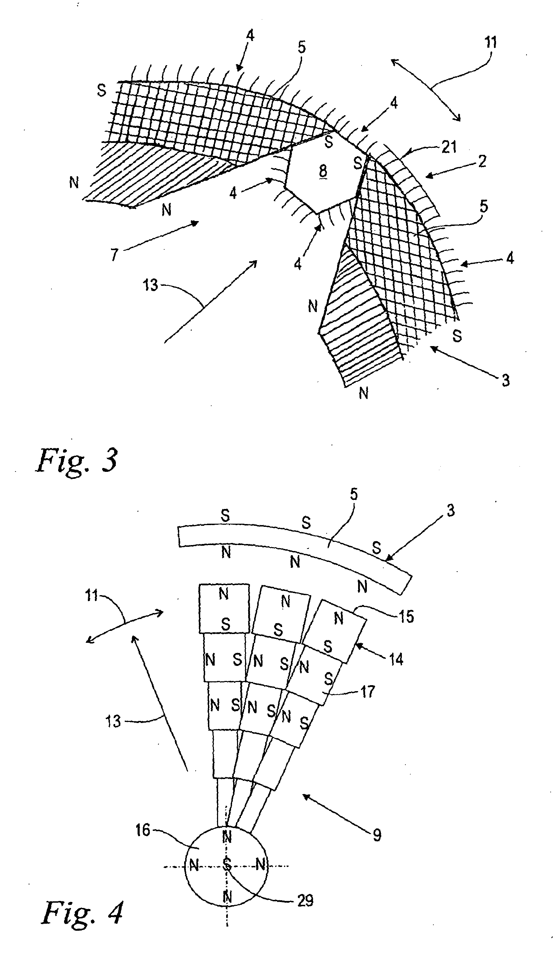

Der Gefäßreiniger 1 ist im wesentlichen zylindrisch aufgebaut, wobei seine Umfangswand an einer Innenwand 21 des Blutgefäßes 2 entsprechend der Darstellung nach Fig. 3 anliegt. Durch die Zylinderform des Gefäßreinigers 1 ist eine Umfangsrichtung 11, eine Axialrichtung 12 und eine Radialrichtung 13 vorgegeben. Bezogen auf die Radialrichtung 13 innenseitig der Reinigungshülse 3 ist ein nicht näher dargestellter Strömungskanal 6 vorgesehen, der durch den Gefäßreiniger 1 in der Axialrichtung 12 hindurchläuft und einen Blutstrom 10 durchläßt. Turbinen 23 der Versiegelungseinrichtungen 20, 20' sowie ein in Fig. 2 dargestellter Rotor 9 geben den Blutstrom 10 durch den Strömungskanal 6 frei. Beim Durchtritt des Blutstromes 10 durch den Gefäßreiniger 1 entsteht ein Strömungswiderstand, der den Gefäßreiniger 1 in Richtung des Blutstromes 10, jedoch mit im Vergleich dazu langsamerer Geschwindigkeit durch das Blutgefäß 2 bewegt. Die rohrförmige Reinigungshülse 3 ist außenseitig mit hier nicht näher dargestellten mechanischen Reinigungsmitteln 4 versehen, die entsprechend der zeichnerischen Darstellung nach Fig. 3 vorteilhaft als elastisch federnde Borsten ausgebildet sind. Es können auch weiche Schaber oder dergleichen zweckmäßig sein. Die mechanischen Reinigungsmittel 4 befreien infolge der axialen Bewegung des Gefäßreinigers 1 die Innenwand 21 (Fig. 3) des Blutgefäßes 2 von ggf. vorhandenen Kalk-, Hormon- und/oder Blutfettablagerungen.

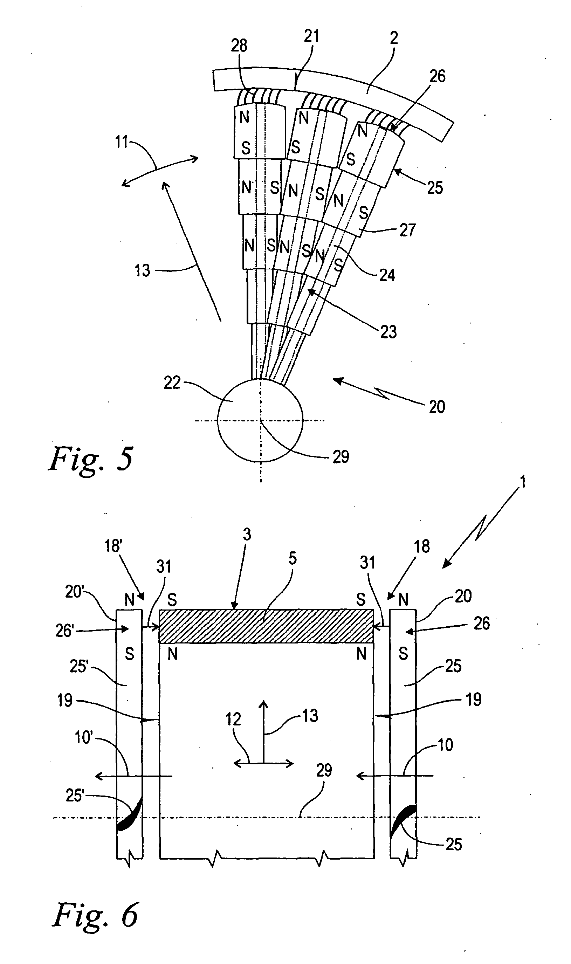

Die beiden Versiegelungseinrichtungen 20, 20' weisen jeweils ein radial inneres Depot 22 für ein Versiegelungsmittel sowie eine Turbine 23 mit in Umfangsrichtung verteilten Turbinenschaufeln 25 auf. Zumindest die Turbine 23, bevorzugt die gesamte Versiegelungseinrichtung 20, 20' ist um eine Drehachse 29 drehbar gelagert. Der durch die Turbine 23 hindurchtretende Blutstrom 10 versetzt die Anordnung in Drehung, wodurch das Versiegelungsmittel vom Depot 22 in der Radialrichtung 13 nach außen zur Innenwand 21 (Fig. 5) des Blutgefäßes 2 gefördert wird. Durch weiter unten näher beschriebene Maßnahmen ist sichergestellt, daß bezogen auf die durch den Blutstrom 10 vorgegebene Bewegungsrichtung des Gefäßreinigers 1 nur die hintere, der Reinigungshülse 3 nachfolgende Versiegelungseinrichtung 20 aktiv ist, während die vorauseilende Versiegelungseinrichtung 20' außer Betrieb genommen ist. Fissuren oder andere Oberflächenbeschädigungen durch den Reinigungsvorgang der Reinigungshülse 3 werden durch das Versiegelungsmittel der nacheilenden Versiegelungseinrichtung 20 ausgeglichen.

Fig. 2 zeigt eine perspektivische Prinzipdarstellung der Reinigungshülse 3 nach Fig. 1. Die Reinigungshülse 3 ist in der Umfangsrichtung 11 in Segmente 5 aufgeteilt, wobei hier beispielhaft vier Segmente 5 gezeigt sind. Es kann auch eine abweichende Anzahl von Segmenten 5 zweckmäßig sein. Die Segmente 5 sind durch in der Axialrichtung 12 verlaufende Spalte 7 voneinander getrennt, wodurch eine selbsttätige Anpassung des Durchmessers der Reingigungshülse 3 an den jeweiligen Durchmesser des Blutgefäßes 2 erzielt wird. Eine Durchmesseranpassung erfolgt durch Aufweitung der Segmente 5 in der Radialrichtung 13 bzw. durch Zusammendrücken entgegen der Radialrichtung 13, wobei sich in der Umfangsrichtung 11 die Breite der Spalte 7 und damit der Abstand der Segmente 5 zueinander entsprechend verändert bzw. einstellt. In der Darstellung nach Fig.2 sind die Segmente 5 in ihrer radial innersten Position gezeigt, wobei die Segmente 5 in der Umfangsrichtung 11 zumindest näherungsweise aneinander anliegen. Der Gesamtdurchmesser der Reinigungshülse 3 bzw. des Gefäßreinigers 1 nach Fig. 1 beträgt hierbei etwa 1 µm für den Betrieb in Gefäßkapillaren. Ausgehend von dieser radial innersten Position ist eine radiale Aufweitung der Segmente 5 um ein Vielfaches des angegebenen Radius vorgesehen, wobei sich die Breite der Spalte 7 in der Umfangsrichtung 11 entsprechend vergrößert. Angestrebt sind mehrere Größenordnungen zwischen dem maximalen und dem minimalen Durchmesser, wobei ein maximaler Durchmesser von bis zu 2 cm erzielt werden soll.

Zur Auffüllung der Spalte 7 bei größer werdendem Spaltmaß ist innenseitig der Reinigungshülse 3 eine Vielzahl von Füllstäben 8 bevorratet, die parallel zur Axialrichtung 12 liegen. Bevorzugt sind die Füllstäbe 8 prismatisch ausgebildet, können jedoch auch eine abweichende Gestalt aufweisen.

Radial innenseitig der Reinigungshülse 3 ist im Strömungskanal 6 ein Rotor 9 mit einer Nabe 16 und mit daran befestigten Rotorschaufeln 14 angeordnet. Radial äußere Spitzen 15 der Rotorschaufeln 14 weisen in der Radialrichtung 13 einen Abstand zu den Segmenten 5 auf. Hierdurch ist ein Ringraum gebildet, in dem die Füllstäbe 8 gelagert sind. Der Durchtritt des Blutstroms 10 durch den Strömungskanal 6 und damit durch den Rotor 9 versetzt diesen in Drehung um die Drehachse 29. Der sich drehende Rotor 9 verleiht dem Blutstrom 10 einen Drall, der seinerseits die Füllstäbe 8 in der Umfangsrichtung 11 bewegt, bis sich diese selbsttätig in die geöffneten Spalte 7 einlagern und damit die Spalte 7 verschließen. Anstelle eines drehenden Rotors 9 können auch feststehende Leitflächen oder dgl. vorgesehen sein, die dem Blutstrom 10 einen Drall verleihen und damit die gewünschte Wirkung auf die Füllstäbe 8 herbeiführen.

In Fig. 2 ist der Bereich eines Spaltes 7 als Einzelheit III markiert, die in vergrößerter Detaildarstellung in Fig. 3 gezeigt ist. Der Spalt 7 ist in der Umfangsrichtung 11 leicht geöffnet und durch einen Füllstab 8 zumindest näherungsweise ausgefüllt. Der Füllstab 8 weist beispielhaft einen Querschnitt in Form eines gleichseitigen und gleichwinkligen Sechsecks auf. Der Spalt 7 ist in der Umfangsrichtung 11 durch Stoßflächen der angrenzenden Segmente 5 begrenzt, wobei die Stoßflächen gegenüber der Radialrichtung 13 einen Winkel von 30° aufweisen. Insgesamt erzeugen die Stoßflächen also eine keilförmige Öffnung des Spaltes 7 entgegen der Radialrichtung 13 nach innen mit einem Öffnungswinkel von 60°. Dies führt dazu, daß der sechseckige Querschnitt des einen oder mehrerer Füllstäbe 8 eine flächige Anlage an den Stoßflächen der Segmente 5 erzeugt. Der Spalt 7 kann zumindest nahezu lückenfrei aufgefüllt werden. Sofern sich der Innendurchmesser des Blutgefäßes 2 entlang der Bewegungsbahn der Reinigungshülse 3 verringert, drückt die Innenwand 21 des Blutgefäßes 2 die Segmente 5 entgegen der Radialrichtung 13 nach innen, wobei sich der Spalt 7 in der Umfangsrichtung 11 und damit einhergehend der Abstand zwischen den Segmenten 5 in der Umfangsrichtung 11 verringert. Die keilförmige Öffnung des Spaltes 7 entgegen der Radialrichtung 13 bewirkt, daß die im Spalt 7 liegenden Füllstäbe 8 entgegen der Radialrichtung 13 nach innen gedrückt werden, wobei sie ihre Vorratsposition radial innenseitig der Segmente 5 entsprechend der Darstellung nach Fig. 2 wieder einnehmen.

Der Darstellung nach Fig. 3 ist noch zu entnehmen, daß die Segmente 5 aus einem permanentmagnetischen, körperverträglichen Material gebildet sind, wobei die Segmente 5 eine untereinander gleiche, in der Radialrichtung 13 verlaufende magnetische Polarisierungsrichtung aufweisen. Im gezeigten Ausführungsbeispiel liegen die mit S bezeichneten Südpole radial außen, während die mit N bezeichneten Nordpole radial innen liegen. Bezogen auf die Umfangsrichtung 11 liegen sich jeweils gleichnamige Pole der beiden aneinander grenzenden Segmente 5 gegenüber, so daß eine magnetische Abstoßungskraft in der Umfangsrichtung 11 zwischen den benachbarten Segmenten 5 wirkt. Die in der Umfangsrichtung 11 wirkenden magnetischen Abstoßungskräfte führen dazu, daß die Segmente 5 bestrebt sind, in der Umfangsrichtung 11 einen größeren Abstand zueinander einzunehmen. Infolge dessen stellt sich bedarfsweise selbsttätig ein vergrößerter Durchmesser der Reinigungshülse 3 ein. Bei einem entlang der Bewegungsbahn der Reinigungshülse 3 sich vergrößernden Durchmesser des Blutgefäßes 2 paßt sich der Durchmesser der Reinigungshülse 3 selbsttätig an den Durchmesser des Blutgefäßes 2 an, indem er selbst größer wird. Die beispielhaft als elastische Borsten ausgeführten mechanischen Reinigungsmittel 4 auf der äußeren Umfangsseite der Segmente 5 werden dadurch gegen die Innenwand 21 des Blutgefäßes 2 unabhängig von dessen Durchmesser angedrückt und können so ihre Reinigungswirkung ausüben. Auch die Füllstäbe 8 sind außenseitig mit mechanischen Reinigungsmitteln 4, hier ebenfalls in Form von elastisch nachgiebigen Borsten versehen. Unabhängig von der Anzahl der im Spalt 7 befindlichen Füllstäbe 8 sind mechanische Reinigungsmittel 4 auch im Bereich der Spalte 7 außenseitig wirksam. An den Kontaktflächen der Füllstäbe 8 untereinander bzw. mit den Stoßflächen der Segmente 5 legen sich die nachgiebigen Borsten flach an die Oberfläche der Füllstäbe 8 an, können sich jedoch bei einer Lageänderung selbsttätig wieder aufrichten.

Die Füllstäbe 8 sind aus einem weichmagnetischen Material gefertigt, wodurch sie die magnetische Polarisierung der angrenzenden Segmente 5 annehmen. Abhängig von der Breite des Spaltes 7 in der Umfangsrichtung 11 kann eine mehr oder weniger große Anzahl von Füllstäben 8 in den Spalt 7 gelangen und diesen ausfüllen, wobei die magnetischen Eigenschaften der Segmente 5 übernommen und weitergeleitet werden. Je nach Anzahl der Füllstäbe 8 ist dies zwar mit einer Abschwächung des Magnetfeldes verbunden. Innerhalb gewisser Grenzen reicht dieses jedoch aus, die in der Umfangsrichtung 11 wirkenden Abstoßungskräfte aufrechtzuerhalten, wodurch die vorstehend beschriebene selbsttätige Größenanpassung in einem großen Durchmesserbereich möglich ist.

Fig. 4 zeigt eine schematische Ausschnittsdarstellung des Rotors 9 nach Fig.2 in einer Stirnansicht. Die gemeinsam mit den Rotorschaufeln 14 um die Drehachse 29 drehbare Nabe 16 ist als in der Radialrichtung 13 polarisierter Permanentmagnet ausgeführt, wobei die Polarisierungsrichtung entgegengesetzt zur magnetischen Polarisierungsrichtung der Segmente 5 ist. Im gezeigten Ausführungsbeispiel liegen hierzu Südpole S radial innen, während Nordpole N radial außenseitig der Nabe 16 angeordnet sind. Die radial äußeren Nordpole N der Nabe 16 erfahren von den radial inneren Nordpolen N der Segmente 5 eine Abstoßungskraft, die den Rotor 9 innenseitig der Reinigungshülse 3 zentriert. Außerdem wird eine frei drehende, magnetische Lagerung des Rotors 9 gebildet.

Die Rotorschaufeln 14 weisen in der Radialrichtung 13 einen selbsttätig einstellbaren Radius auf. Hierzu sind diese beispielhaft aus einer Anzahl von Teleskopstäben mit Teleskopelementen 17 gebildet, die in der Radialrichtung 13 aus- und einfahrbar sind. Die mittleren und radial inneren Teleskopsegmente 17 sind quer zur Radialrichtung 13, also in der Umfangsrichtung 11 und auch in der Axialrichtung 12 (Fig.2) permanentmagnetisch polarisiert. Hierdurch haften die einzelnen Teleskopstäbe magnetisch aneinander. Die Summe aller magnetisch aneinander haftenden Teleskopstäbe bilden die Rotorschaufeln 14 mit einem geeigneten hydrodynamischen Qurschnitt bzw. Profil.

Nicht nur die Nabe 16 und die radial inneren Teleskopelemente 17, sondern auch die radial äußeren Teleskopelemente 17 im Bereich der Spitzen 15 sind aus einem permanentmagnetischen Material gebildet. Die Teleskopelemente 17 im Bereich der Spitzen 15 weisen ebenso wie die Nabe 16 eine magnetische Polarisierungsrichtung auf, die der magnetischen Polarisierung der Segmente 5 entgegengesetzt ist. Hierdurch entstehen in der Radialrichtung 13 Abstoßungskräfte zwischen den Spitzen 15 und den angrenzenden Segmenten 5, die bestrebt sind, die Spitzen 15 radial nach innen zu drücken.

Die bezogen auf die Radialrichtung 13 mittleren und inneren Teleskopsegmente 17 erzeigen in der Radialrichtung 13 untereinander eine Abstoßungskraft, da in der Radialrichtung 13 jeweils gleichnamige Pole aneinander grenzen. Die aus den Teleskopelementen 17 gebildeten Teleskopstäbe sind demnach bestrebt, selbsttätig in der Radialrichtung 13 auszufahren und damit den Durchmesser des Rotors 9 zu vergrößern. Dieser Effekt wird noch unterstützt durch die bei einer Drehung um die Drehachse 29 wirkenden Fliehkräfte. Die vorgenannte radiale Aufweitung bzw. selbsttätige Vergrößerung ist jedoch begrenzt durch die Abstoßungskräfte zwischen den Spitzen 15 und den Segmenten 5, so daß immer ein radialer Spalt verbleibt. Bei einer Durchmesserveränderung der Reinigungshülse 3 wird jedoch der Durchmesser des Rotors 9 unter Beibehaltung des radialen Spaltes selbsttätig nachgeführt, also selbsttätig verkleinert bzw. vergrößert.

Fig. 5 zeigt eine schematische Ausschnittsdarstellung der Versiegelungseinrichtung 20 nach Fig. 1 in Stirnansicht, demnach die Turbinenschaufeln 25 der Turbine 23 vergleichbar zum Rotor 9 nach Fig. 4 aus Teleskopsegmenten 27 aufgebaut sind. Die Teleskopsegmente 27 sind entsprechend den Teleskopsegmenten 17 des Rotors 9 nach Fig. 4 aus einem permanentmagnetischen Material gefertigt und in gleicher Weise polarisiert. Die einzelnen aus den Teleskopsegmenten 27 gebildeten Teleskopstäbe haften unter Bildung der Turbinenschaufeln 25 magnetisch aneinander. Im übrigen sind sie bestrebt, selbsttätig ihren Radius und damit den Durchmesser der Turbine 23 zu vergrößern. Bezüglich Einzelheiten der vorgenannten magnetischen und fliehkraftbedingten Wirkung wird auf die diesbezüglich vergleichbare Beschreibung zur Anordnung nach Fig. 4 verwiesen.

Abweichend von der Anordnung nach Fig. 4 sind radial äußere Spitzen 26 der Turbinenschaufeln 25 gegen die Innenwand 21 des Blutgefäßes 2 abgestützt. Mit größer werdendem Durchmesser des Blutgefäßes 2 vergrößert sich auch der Durchmesser der Turbine 23 selbsttätig, wobei die äußeren Spitzen 26 an der Innenwand 21 anliegend bleiben. Bei enger werdendem Durchmesser des Blutgefäßes 2 drückt dessen Innenwand 21 die einzelnen Teleskopsegmente 27 entgegen der Radialrichtung 13 ineinander. Insgesamt ist ein selbsttätig einstellbarer Radius bzw. Durchmesser der Turbine 23 mit seinen Turbinenschaufeln 25 erzeugt.

Innenseitig der Teleskopstäbe verlaufen in der Radialrichtung 13 Förderkanäle 24 für das im Depot 22 bevorratete Versiegelungsmittel. Die bei Drehung der Turbine 23 um die Drehachse 29 wirkenden Fliehkräfte fördern das Versiegelungsmittel vom Depot 22 durch die Förderkanäle 24 in der Radialrichtung 13 nach außen zur Innenwand 21 des Blutgefäßes 2. Zur Verteilung und zur Anbringung des Versiegelungsmittels an der Innenwand 21 ist an den Spitzen 26 der Turbinenschaufeln 25 eine Anzahl von weichen Hohlborsten 28 angeordnet, durch die das Versiegelungsmittel ausgehend von den Förderkanälen 24 hindurchgeführt und auf die Innenwand 21 aufgebracht wird. Zur Schonung der Innenwand 21 sind die Turbinenschaufeln 25 radial außenseitig an den Spitzen 26 gerundet ausgeführt.

Der Darstellung nach Fig. 5 ist noch zu entnehmen, daß abweichend von den radial mittleren und inneren Teleskopsegmenten 27 mit quer zur Radialrichtung 13 verlaufender magnetischer Polarisierung die radial äußeren Teleskopelemente 27 im Bereich der Spitzen 26 in der Radialrichtung 13 magnetisch polarisiert sind, wobei die magnetische Polarisierung entgegengesetzt zur radialen magnetischen Polarisierung der Segmente 5 entsprechend der Darstellung nach Fig. 6 verläuft. Aus der schematischen Längsschnittdarstellung des Gefäßreinigers 1 nach Fig. 6 ergibt sich das magnetische Wechselspiel zwischen der Reinigungshülse 3 und den Turbinenschaufeln 25, 25' der beiden Versiegelungseinrichtungen 20, 20'.

Die beiden Versiegelungseinrichtungen 20, 20' sind identisch zueinander aufgebaut und spiegelsymmetrisch an den beiden Stirnseiten 19, 19' angeordnet. Die beiden Turbinen 23, 23' sind um die gleiche Drehachse 29 wie der Rotor 9 (Fig. 2) drehbar, wobei jedoch jeweils eine eigenständige, voneinander unabhängige Drehung zugelassen ist. Die Turbinen 23, 23' sind zusammen mit dem Rotor 9 (Fig. 2) auf einer nicht dargestellten, gemeinsamen Achse gelagert. Die Zentrierung der Turbinen 23, 23' erfolgt über die Zentrierung der Nabe 16 (Fig. 4).

Da entsprechend der vorangegangenen Beschreibung sowohl der Außendurchmesser der Reinigungshülse 3 als auch der Außendurchmesser der beiden Versiegelungseinrichtungen 20, 20' durch den Innendurchmesser des Blutgefäßes 2 (Fig. 4, 5) bestimmt wird, sind beide Durchmesser etwa gleich groß. Die stirnseitige Anordnung der beiden Versiegelungseinrichtungen 20, 20' an der Reinigungshülse 3 bewirkt, daß die Spitzen 26, 26' der Turbinenschaufeln 25, 25' in der Axialrichtung 12 an entgegengesetzte magnetische Pole der Reinigungshülse 3 angrenzen. Dies erzeugt in der Axialrichtung 12 eine Anziehungskraft zwischen den Spitzen 26, 26' und der Reinigungshülse 3. Die Spitzen 26, 26' legen sich an die Stirnflächen der Reinigungshülse 3 an. Es kann zweckmäßig sein, bezogen auf die Axialrichtung 12 zwischen den Turbinenschaufeln 25, 25' und der Reinigungshülse 3 einen axialen Abstand zu belassen. Zumindest oberhalb eines bestimmten eingestellten Durchmessers der Anordnung ist die Flexibilität der Turbinenschaufeln 25, 25' jedoch so groß, daß sich die Spitzen 26, 26' entsprechend Pfeilen 31 an die Stirnseiten der Reinigungshülse 3 elastisch federnd anlegen. Bei geeigneter Auslegung der Versiegelungseinrichtungen 20, 20' kann jedoch das vom durchtretenden Blutstrom 10 an den Turbinenschaufeln 25, 25' um die Drehachse 29 wirkende Drehmoment ausreichen, die magnetischen Anpreßkräfte an den Spitzen 26, 26' zu überwinden, so daß eine Drehung möglich ist.

In der erfindungsgemäßen Ausgestaltung ist vorgesehen, daß nur die in der durch den Blutstrom 10 vorgegebenen Bewegungsrichtung nacheilende Versiegelungseinrichtung 20 gedreht wird, während die der Reinigungshülse 3 vorauseilende Versiegelungseinrichtung 20' stillsteht. Dies kann durch mehrere Effekte für sich alleine oder in Kombination erzielt werden. Der eintretende Blutstrom 10 weist eine höhere Energie auf als der austretende Blutstrom 10'. Durch geeignete Abstimmung der magnetischen Andruckkräfte an den Spitzen 26, 26' kann erzielt werden, daß die Energie des eintretenden Blutstromes 10 ausreicht, die nachlaufende Versiegelungseinrichtung 20 in Drehung zu versetzen, während die magnetische Anpreßkraft an den Spitzen 26' der vorauseilenden Versiegelungseinrichtung 20' durch den austretenden Blutstrom 10' mit verringerter Energie nicht überwunden werden kann.

Der Darstellung nach Fig. 6 kann entnommen werden, daß die beiden Versiegelungseinrichtungen 20, 20' identisch zueinander ausgebildet sind und spiegelsymmetrisch zueinander an den Stirnseiten 19, 19' der Reinigungshülse 3 angeordnet sind.

Jeweils eine der Turbinenschaufeln 25, 25' ist im Querschnitt gezeigt, demnach sie ein hydrodynamisches, gewölbtes Tropfenprofil aufweisen. Das Profil ist derart angeordnet, daß es für die Strömungsverhältnisse des eintretenden Blutstromes 10 angepaßt ist und dabei seine optimale strömungstechnische Wirkung entfaltet. Infolge der spiegelsymmetrischen Anordnung wird das Profil der Turbinenschaufel 25' auf der Vorderseite der Anordnung durch den austretenden Blutstrom 10' in entgegengesetzter, also nicht optimaler Richtung angeströmt, wodurch seine hydrodynamische Wirkung erheblich verringert ist. Dies führt dazu, daß nur die eingangsseitige, also bezogen auf die Bewegungsrichtung 10 nacheilende Versiegelungseinrichtung 20 gedreht wird, während die ausgangseitige, vorauseilende Versiegelungseinrichtung 20' stillsteht oder sich zumindest nur eingeschränkt bewegt.

Alternativ oder unterstützend können noch mechanische Blockiereinrichtungen vorgesehen sein. Beispielsweise ist es möglich, die beiden Versiegelungseinrichtungen 20, 20' auf einer gemeinsamen Nabenanordnung anzuordnen, die in der Axialrichtung 12 gegenüber der Reinigungshülse 3 infolge des anliegenden Staudruckes verschiebbar ist. Infolge des Verschiebeweges kann die ausgangsseitige Verssiegelungseinrichtung 20' blockiert werden, während die eingangsseitige Versiegelungseinrichtung 20 freigegeben ist.

Den vorstehenden Ausführungen kann insgesamt entnommen werden, daß es infolge der spiegelsymmetrischen Ausbildung des Gefäßreinigers 1 nicht auf dessen Bewegungsrichtung innerhalb des Blutgefäßes 2 (Fig. 1) ankommt. Bei einer Bewegung in der Axialrichtung 12, jedoch entgegengesetzt zur Darstellung nach Fig. 6 erfolgt eine Durchströmung entgegen der Pfeile 10, 10', wodurch sich die Verhältnisse umkehren. Die vormals ausgangsseitige Versiegelungseinrichtung 20' liegt nun eingangsseitig und wird in vorstehender Weise angetrieben, während die vormals eingangsseitige Versiegelungseinrichtung 20 nun auf der Austrittsseite liegt, dadurch blockiert und nicht mehr aktiv ist.

Unter gleichzeitigem Bezug auf die Fig. 5 und 6 ist hervorzuheben, daß die Teleskopelemente 27 zwar in der Radialrichtung 13 frei gegeneinander verschiebbar sind. Der Verschiebeweg ist jedoch radial nach außen durch nicht dargestellte Anschläge begrenzt. Es kann nur ein vorgegebener maximaler Durchmesser erreicht werden. Sofern der Innendurchmesser des Blutgefäßes 2 diesen maximalen Außendurchmesser überschreitet - insbesondere beim Eintritt des Gefäßreinigers in eine Herzkammer - fehlt die Stützwirkung der Innenwand 21 des Blutgefäßes 2. Der Radius der Turbinenschaufelns 25 vergrößert sich dabei jedoch nicht beliebig, sondern ist durch die vorgenannten Anschläge begrenzt.

Hierbei haften die Spitzen 26, 26' der Turbinenschaufeln 25, 25' an den Stirnseiten der Reinigungshülse 3 infolge ihrer magnetischen Anziehungskraft. Die Segmente 5 können sich beim Fehlen der Stützwirkung der Innenwand 21 infolge der vorgenannten Haltekraft nur bis zum maximalen Radius bzw. Durchmesser der Turbinenschaufeln 25, 25' aufweiten. Jede der beiden Versiegelungseinrichtungen 20, 20' bildet dadurch eine radial aufweitbare, permanentmagnetisch polarisierte Begrenzungseinrichtung 18 zur Begrenzung der radialen Aufweitung der Segmente 5. Es kann aber auch zweckmäßig sein, die Begrenzungseinrichtungen 18, 18' nicht durch die Turbinenschaufeln 25, 25' zu bilden, sondern eine funktionale Trennung mit separaten Begrenzungseinrichtungen 18, 18' vorzusehen.

Die Versiegelungseinrichtungen 20, 20' üben darüber hinaus noch eine Sicherungsfunktion für die Füllstäbe 8 (Fig. 2) aus. Die in Fig. 6 nicht dargestellten Füllstäbe 8 erstrecken sich in der Axialrichtung 12 über die gesamte Länge der Reinigungshülse 3, wobei sie durch die beiden stirnseitig angeordneten Versiegelungseinrichtungen 20, 20' an einem Verrutschen bzw. an einem Herausfallen in der Axialrichtung 12 gehindert werden.

## Patentansprüche

1. Frei in der Blutbahn beweglicher Gefäßreiniger (1) zur Reinigung von Blutgefäßen (2), vorgesehen zur Bewegung im Blutgefäß (2) infolge seines beim Durchtritt des Blutstromes entstehenden Durchströmungswiderstandes, umfassend eine rohrförmige Reinigungshülse (3), die außenseitig mit mechanischen Reinigungsmitteln (4) versehen ist, wobei die Reinigungshülse (3) bezogen auf eine Umfangsrichtung (11) des Gefäßreinigers (1) in Segmente (5) aufgeteilt ist, wobei die Segmente (5) in der Umfangsrichtung (11) im Abstand zueinander einstellbar sind, so dass eine Anpassung an verschiedene Gefäßdurchmesser erlaubt ist, und wobei der Gefäßreiniger (1) einen in seiner Axialrichtung (12) verlaufenden Strömungskanal (6) für Blut aufweist.

2. Gefäßreiniger nach Anspruch 1,
**dadurch gekennzeichnet, daß** Spalte (7) zwischen den Segmenten (4) der Reinigungshülse (3) keilförmig in einer Radialrichtung (13) nach innen geöffnet ausgebildet sind, und daß insbesondere prismatische Füllstäbe (8) zur Auffüllung der Spalte (7) innenseitig der Reinigungshülse (3) bevorratet sind.

3. Gefäßreiniger nach Anspruch 1 oder 2,
**dadurch gekennzeichnet, daß** die Segmente (5) aus einem permanentmagnetischen Material gebildet sind, wobei die Segmente (5) eine untereinander gleiche, in der Radialrichtung (13) des Gefäßreinigers (1) verlaufende magnetische Polarisierungsrichtung aufweisen.

4. Gefäßreiniger nach den Ansprüchen 2 und 3,
**dadurch gekennzeichnet, daß** die Füllstäbe (8) aus einem weichmagnetischen Material sind.

5. Gefäßreiniger nach einem der Ansprüche 1 bis 4,
**dadurch gekennzeichnet, daß** die Füllstäbe (8) außenseitig mit elastisch verformbaren mechanischen Reinigungsmitteln (4) versehen sind.

6. Gefäßreiniger nach einem der Ansprüche 1 bis 5,
**dadurch gekennzeichnet, daß** im Strömungskanal (6) ein Rotor (9) angeordnet ist, zu dessen Antrieb der durchtretende Blutstrom (10) vorgesehen ist.

7. Gefäßreiniger nach Anspruch 6,
**dadurch gekennzeichnet, daß** der Rotor (9) zumindest teilweise aus einem permanentmagnetischen Material gebildet ist, wobei zumindest Spitzen (15) von Rotorschaufeln (14) des Rotors (9), bevorzugt auch eine Nabe (16) des Rotors (9) eine in der Radialrichtung (13) verlaufende magnetische Polarisierungsrichtung aufweisen, die der magnetischen Polarisierung der Segmente (5) entgegengesetzt ist.

8. Gefäßreiniger nach Anspruch 6 oder 7,
**dadurch gekennzeichnet, daß** der Rotor (9) Rotorschaufeln (14) mit selbsttätig einstellbarem Radius aufweist, die bevorzugt aus in der Radialrichtung (13) angeordneten Teleskopelementen (17) gebildet sind, wobei zumindest ein Teil der Teleskopelemente (17) quer zur Radialrichtung (13) permanentmagnetisch polarisiert ist.

9. Gefäßreiniger nach einem der Ansprüche 1 bis 8,
**dadurch gekennzeichnet, daß** stirnseitig der Reinigungshülse (3) eine radial aufweitbare, insbesondere permanentmagnetisch polarisierte Begrenzungseinrichtung (18) zur Begrenzung einer radialen Aufweitung der Segmente (5) vorgesehen ist.

10. Gefäßreiniger nach einem der Ansprüche 1 bis 9,
**dadurch gekennzeichnet, daß** auf einer bezogen auf die Bewegungsrichtung rückwärtigen Stirnseite (19) der Reinigungshülse (3) eine Versiegelungseinrichtung (20) für eine Innenwand (21) des Blutgefäßes (2) angeordnet ist, wobei die Versiegelungseinrichtung (20) ein radial inneres Depot (22) für ein Versiegelungsmittel sowie eine zum Antrieb vom durchtretenden Blutstrom (10) vorgesehene Turbine (23) umfasst, und wobei die Turbine (23) in der Radialrichtung (13) verlaufende Förderkanäle (24) für das Versiegelungsmittel aufweist.

11. Gefäßreiniger nach Anspruch 10,
**dadurch gekennzeichnet, daß** die Turbine (23) zumindest teilweise aus einem permanentmagnetischen Material gebildet ist, wobei zumindest Spitzen (26) von Turbinenschaufeln (25) der Turbine (23) eine in der Radialrichtung (13) verlaufende magnetische Polarisierungsrichtung aufweisen, die der magnetischen Polarisierung der Segmente (5) entgegengesetzt ist.

12. Gefäßreiniger nach Anspruch 10 oder 11,
**dadurch gekennzeichnet, daß** die Turbine (23) Turbinenschaufeln (24) mit selbsttätig einstellbarem Radius aufweist, die bevorzugt aus in der Radialrichtung (13) angeordneten Teleskopelementen (27) gebildet sind, wobei zumindest ein Teil der Teleskopelemente (27) quer zur Radialrichtung (13) permanentmagnetisch polarisiert ist.

13. Gefäßreiniger nach den Ansprüchen 9 und 12,
**dadurch gekennzeichnet, daß** die Begrenzungseinrichtung (18) durch die Turbinenschaufeln (25) gebildet ist.

14. Gefäßreiniger nach einem der Ansprüche 10 bis 13,
**dadurch gekennzeichnet, daß** die Turbinenschaufeln (25) radial außenseitig gerundet ausgeführt sind und dort Hohlborsten (28) zur Anbringung des Versiegelungsmittels an die Innenwand (21) des Blutgefäßes (2) aufweisen.

15. Gefäßreiniger nach einem der Ansprüche 10 bis 14,
**dadurch gekennzeichnet, daß** zwei im wesentlichen identisch ausgebildete Versiegelungseinrichtungen (20, 20') an je einer Stirnseite (19, 19') der Reinigungshülse (3) spiegelsymmetrisch zueinander angeordnet sind.

## Claims

1. Vessel cleaner (1), which can move freely in the bloodstream, for cleaning blood vessels (2), provided for movement in the blood vessel (2) owing to its flow resistance, which arises when it passes through the bloodstream, comprising a tubular cleaning sleeve (3), which is provided on the outside with mechanical cleaning means (4), wherein the cleaning sleeve (3) is divided into segments (5) in relation to a circumferential direction (11) of the vessel cleaner (1), wherein the spacing of the segments (5) with respect to each other can be adjusted in the circumferential direction (11) so that an adaptation to different vessel diameters is allowed, and wherein the vessel cleaner (1) has a flow duct (6), which runs in its axial direction (12), for blood.

2. Vessel cleaner according to Claim 1,
**characterised in that** gaps (7) between the segments (4) of the cleaning sleeve (3) are configured such that they open inwardly in a radial direction (13) in a wedge-shaped manner, and that in particular prismatic filling rods (8) are kept inside the cleaning sleeve (3) for filling the gaps (7).

3. Vessel cleaner according to Claim 1 or 2,
**characterised in that** the segments (5) are formed from a permanently magnetic material, wherein the segments (5) have the same magnetic polarisation direction which runs in the radial direction (13) of the vessel cleaner (1).

4. Vessel cleaner according to Claims 2 and 3,
**characterised in that** the filling rods (8) consist of a magnetically soft material.

5. Vessel cleaner according to one of Claims 1 to 4,
**characterised in that** the filling rods (8) are provided on the outside with elastically deformable mechanical cleaning means (4).

6. Vessel cleaner according to one of Claims 1 to 5,
**characterised in that** a rotor (9) is arranged in the flow duct (6), which rotor is driven by the bloodstream (10) which passes through.

7. Vessel cleaner according to Claim 6,
**characterised in that** the rotor (9) is at least partially formed from a permanently magnetic material, wherein at least points (15) of rotor blades (14) of the rotor (9), preferably also a hub (16) of the rotor (9), have a magnetic polarisation direction which runs in the radial direction (13) and is opposed to the magnetic polarisation of the segments (5).

8. Vessel cleaner according to Claim 6 or 7,
**characterised in that** the rotor (9) has rotor blades (14) with an automatically adjustable radius, which blades are preferably formed from telescopic elements (17) which are arranged in the radial direction (13), wherein at least some of the telescopic elements (17) are polarised in a permanently magnetic manner transversely with respect to the radial direction (13).

9. Vessel cleaner according to one of Claims 1 to 8,
**characterised in that** a limiting device (18), which can be radially widened and is polarised in particular in a permanently magnetic manner, for limiting a radial widening of the segments (5) is provided on the end face of the cleaning sleeve (3).

10. Vessel cleaner according to one of Claims 1 to 9,
**characterised in that** a sealing device (20) for an inner wall (21) of the blood vessel (2) is arranged on an end face (19), which is to the rear in relation to the movement direction, of the cleaning sleeve (3), wherein the sealing device (20) comprises a radially inner store (22) for a sealant and a turbine (23) which is provided to drive the bloodstream (10) which passes through, and wherein the turbine (23) has feed ducts (24), which run in the radial direction (13), for the sealant.

11. Vessel cleaner according to Claim 10,
**characterised in that** the turbine (23) is at least partially formed from a permanently magnetic material, wherein at least points (26) of turbine blades (25) of the turbine (23) have a magnetic polarisation direction which runs in the radial direction (13) and is opposed to the magnetic polarisation of the segments (5).

12. Vessel cleaner according to Claim 10 or 11,
**characterised in that** the turbine (23) has turbine blades (24) with an automatically adjustable radius, which blades are preferably formed from telescopic elements (27) which are arranged in the radial direction (13), wherein at least some of the telescopic elements (27) are polarised in a permanently magnetic manner transversely with respect to the radial direction (13).

13. Vessel cleaner according to Claims 9 and 12,
**characterised in that** the limiting device (18) is formed by the turbine blades (25).

14. Vessel cleaner according to one of Claims 10 to 13,
**characterised in that** the turbine blades (25) are designed to be rounded on the radially outer side and have hollow bristles (28) there for applying the sealant to the inner wall (21) of the blood vessel (2) .

15. Vessel cleaner according to one of Claims 10 to 14,
**characterised in that** two essentially identically configured sealing devices (20, 20') are arranged mirror-symmetrically with respect to each other, one at each end face (19, 19') of the cleaning sleeve (3).

## Revendications

1. Purificateur de vaisseaux (1) mobile dans la voie sanguine pour la purification de vaisseaux sanguin (2), prévu pour le déplacement dans le vaisseau sanguin (2) du fait de sa résistance à l'écoulement se formant lors du passage du flux de sang, comprenant un manchon de nettoyage (3) en forme de tube, qui est doté côté extérieur d'agents de nettoyage (4) mécaniques, le manchon de nettoyage (3) étant divisé en segments (5) par rapport à une direction périphérique (11) du purificateur de vaisseaux (1), les segments (5) pouvant être réglés dans la direction périphérique (11) à distance les uns des autres de telle sorte qu'une adaptation à différents diamètres de vaisseau est autorisée et le purificateur de vaisseaux (1) présente un canal d'écoulement (6), agencé dans sa direction axiale (12), pour du sang.

2. Purificateur de vaisseaux selon la revendication 1,
**caractérisé en ce que** des fentes (7) sont conçues entre les segments de (4) du manchon de nettoyage (3), en forme de clavette, dans une direction radiale (13), ouvertes vers l'intérieur et **en ce qu'**en particulier des barres de remplissage (8) prismatiques sont alimentés pour le remplissage des fentes (7) côté intérieur du manchon de nettoyage (3).

3. Purificateur de vaisseaux selon la revendication 1 ou 2,
**caractérisé en ce que** les segments (5) sont formés à base d'un matériau à magnétisme permanent, les segments (5) présentant une direction de polarisation magnétique, identique les uns par rapport aux autres, agencés dans la direction radiale (13) du purificateur de vaisseaux (1).

4. Purificateur de vaisseaux selon les revendications 2 et 3,
**caractérisé en ce que** les barres de remplissage (8) sont à base d'un matériau magnétique doux.

5. Purificateur de vaisseaux selon l'une quelconque des revendications 1 à 4, **caractérisé en ce que** les barres de remplissage (8) sont dotées côté extérieur d'agents de nettoyage (4) mécaniques et élastiquement déformables.

6. Purificateur de vaisseaux selon l'une quelconque des revendications 1 à 5, **caractérisé en ce que** dans le canal d'écoulement (6) est disposé un rotor (9), pour l'entraînement duquel le flux de sang (10) passant est prévu.

7. Purificateur de vaisseaux selon la revendication 6,
**caractérisé en ce que** le rotor (9) est formé au moins en partie à base d'un matériau à magnétisme permanent, au moins des pointes (15) de pales de rotor (14) du rotor (9), de préférence également un moyeu (16) du rotor (9) présentant une direction de polarisation magnétique agencée dans la direction radiale (13), qui est opposée à la polarisation magnétique des segments (5).

8. Purificateur de vaisseaux selon la revendication 6 ou 7,
**caractérisé en ce que** le rotor (9) présente des pales de rotor (14) avec un rayon réglable automatiquement, lesquels sont formés de préférence à partir d'éléments télescopiques (17) disposés dans la direction radiale (13), au moins une partie des éléments télescopiques (17) étant polarisée de façon magnétique en permanence transversalement à la direction radiale (13).

9. Purificateur de vaisseaux selon l'une quelconque des revendications 1 à 8, **caractérisé en ce que**, sur le côté avant du manchon de nettoyage (3), il est prévu un dispositif de délimitation (18) élargi radialement, en particulier polarisé avec un magnétisme permanent, pour délimiter un élargissement radial des segments (5).

10. Purificateur de vaisseaux selon l'une quelconque des revendications 1 à 9, **caractérisé en ce qu'**un dispositif de scellement (20) pour une paroi intérieure (21) du vaisseau sanguin (2) est disposé sur un côté avant (19), arrière par rapport au sens de déplacement, du manchon de nettoyage (3), le dispositif de scellement (20) comportant un dépôt (22) radialement intérieur pour un moyen de scellement et une turbine (23) prévue pour l'entraînement de flux de sang (10) passant, et la turbine (23) présentant des cadeaux de transport (24) agencés dans la direction radiale (13) pour le moyen de scellement.

11. Purificateur de vaisseaux selon la revendication 10, **caractérisé en ce que** la turbine (23) est formée au moins en partie à base d'un matériau à magnétisme permanent, au moins les pointes (26) de pales de turbine (25) de la turbine (23) présentant une direction de polarisation magnétique agencée dans la direction radiale (13), qui est opposée à la polarisation magnétique des segments (5).

12. Purificateur de vaisseaux selon la revendication 10 ou 11,
**caractérisé en ce que** la turbine (23) présente des pales de turbine (24) avec un rayon réglable automatiquement, lesquelles sont formées de préférence à partir d'éléments télescopiques (27) disposés dans la direction radiale (13), au moins une partie des éléments télescopiques (27) étant polarisés avec un magnétisme permanent transversalement à la direction radiale (13).

13. Purificateur de vaisseaux selon les revendications 9 et 12, **caractérisé en ce que** le dispositif de délimitation (18) est formé par les aubes de turbine (25).

14. Purificateur de vaisseaux selon l'une quelconque des revendications 10 à 13, **caractérisé en ce que** les aubes de turbine (25) sont réalisées de façon arrondie radialement côté extérieur et présentent à cet endroit des crins creux (28) pour le placement de l'agent de scellement sur la paroi intérieure (21) du vaisseau sanguin (2).

15. Purificateur de vaisseaux selon l'une quelconque des revendications 10 à 14, **caractérisé en ce que** deux dispositifs de scellement (20, 20') conçus sensiblement identiques sont disposés chacun sur un côté avant (19, 19') du manchon de nettoyage (3) de façon symétrique l'un par rapport à l'autre.
